# EUROPEAN PATENT APPLICATION

(11) **EP 3 539 615 A1**
(43) Date of publication of application: **18.09.2019**
(21) Application number: 17869663.9
(22) Date of filing: 06.11.2017
(51) Int. Cl.: A61N 5/06, A61N 5/067

(54) **RHINITIS THERAPEUTIC DEVICE AND METHOD FOR CONTROLLING RHINITIS THERAPEUTIC DEVICE**

(30) Priority: 14.11.2016 KR 20160151433
(71) Applicant: Mirint Co., Ltd, Seongnam-si, Gyeonggi-do 13174 (KR)
(72) Inventor: KIM, Yong Ho, Seongnam-si Gyeonggi-do 13174 (KR)
(74) Representative: Potter Clarkson
(86) International application number: PCT/KR2017/012498
(87) International publication number: WO 2018/088767

(57) **Abstract**

The present invention relates to a rhinitis therapeutic device including a main body and a pair of insertion rods, and provides a rhinitis thrapeutic device and a control method thereof for preventing a light source from irradiating light irrespective of a user's intention during use. A sensing light source and a light receiver are installed on inner surfaces of insertion rods of the rhinitis therapeutic device. The sensing light source emits light when power is supplied. An intensity of light of the sensing light source is preferably of a low intensity which is less likely to cause eye damage. The light from the sensing light source is detected in the light receiver, and when the rhinitis therapeutic device is inserted into the nose, the light of the sensing light source is blocked by the nasal septum. At least one therapeutic light source formed on the pair of insertion rods is controlled to emit light when the amount of light detected by the light receiver is less than or equal to a predetermined reference light amount or to emit light after power is supplied and then a predetermined reference time elapses. Thus, it is possible to reduce the possibility of eye damage by the light source of the rhinitis therapeutic device.

## Description

### [Technical Field]

The present invention relates to a rhinitis therapeutic device, and more particularly, to a rhinitis therapeutic device using light and a control method thereof.

### [Background Art]

Environmental factors such as increase of fine dust are increasing the number of patients with rhinitis. Various methods for treating rhinitis have been proposed, one of which is using light. For a rhinitis therapeutic device using light, Korean Patent Publication No. 10-0987729 discloses a rhinitis therapeutic device using an LED, Korean Patent Laid-Open Publication No. 10-2014-0087195 discloses a control application for therapeutic of rhinitis, and a portable communication terminal based on Android operation using the same, and Korean patent Publication No. 10-0977985 discloses a multi-wavelength laser irradiator for treating rhinitis.

All of these rhinitis therapeutic devices are intended to treat rhinitis by irradiating light. However, since the nose is located at a position very close to the eyes, if light to be irradiated for treating the rhinitis is directly irradiated to the eyes, the eyes may be damaged. In addition, in the process of controlling the rhinitis therapeutic device, there is a possibility of damaging the eyes such as when a switch of the light is turned on and the light is directly irradiated to the eyes in a state where it is not inserted into the nose.

### [DISCLOSURE]

### [Technical Problem]

An object of the present invention is to provide a rhinitis therapeutic device and a control method thereof, capable of reducing the possibility of damage to eyes adjacent to the nose by unintentional light irradiation in the course of using the rhinitis therapeutic device using light.

### [Technical Solution]

An aspect of the present invention provides a rhinitis therapeutic device including: a power source; a main body configured to accommodate the power source; a pair of insertion rods coupled to the body and configured to be inserted into the nose for therapy; at least one sensing light source; at least one light receiver; at least one therapeutic light source formed in the pair of insertion rods; and a controller accommodated in the main body to be coupled to the power source, said at least one sensing light source, said at least one light receiver, and said at least therapeutic light source, said at least one sensing light source is formed on an inner surface of a first one of the pair of insertion rods, and said at least one light receiver is formed on an inner surface of a second one of the pair of insertion rods, to correspond to said at least one sensing light source to receive light from the sensing light source, and the controller controls the sensing light source to emit light when the power source is on, and controls said at least one therapeutic light source to emit light when the power is on and an amount of light detected by the light receiver is less than or equal to a predetermined reference light amount. Herein, the meaning that the power is turned on indicates that a user starts rhinitis therapy.

Said at least one therapeutic light source may be at least one laser diode, may be at least one LED, and may be a combination of the laser diode and the LED. In an exemplary embodiment, the sensing light source may output light in a predetermined first light intensity when the amount of light detected by the light receiver is less than or equal to the reference light amount and may output light in a predetermined light intensity when the amount of light detected by the light receiver is larger than the reference light amount, and the first light intensity is lower than the second light intensity. In an exemplary embodiment, the rhinitis therapeutic device may further include a power switch for turning the power source on/off. The rhinitis therapeutic device may further include a communication module, and a signal for turning on/off the power source may be received from an external device through the communication module, and is transmitted to the controller. In an exemplary embodiment, the rhinitis therapeutic device may further include a power indicator formed at a side of the main body opposite to a side at which the pair of insertion rods are formed. The power indicator may include a plurality of LED display elements, and the controller may control the LED display elements to emit light continuously or in a predetermined pattern when the power is on

In an exemplary embodiment, light output from the sensing light source may be emitted in an extended form obliquely and/or in a direction that is substantially perpendicular to a longitudinal direction of the pair of insertion rods

This is intended to compensate for manufacturing tolerances or deviations that may occur in use.

An aspect of the present invention provides a rhinitis therapeutic device including: a main body; a power switch formed on the main body to turn on/off the power by a user operation; a pair of insertion rods coupled to the body and configured to be inserted into the nose for therapy; at least one therapeutic light source formed in the pair of insertion rods; and a control unit accommodated in the main body and coupled to said at least one therapeutic light source, wherein the controller receives an on signal from the power switch and then controls said at least one therapeutic light source to emit light after a predetermined reference time elapses. According to an exemplary embodiment, said at least one therapeutic light source may be a laser diode, LED or a combination thereof. In an exemplary embodiment, the rhinitis therapeutic device may further include at least one sensing light source formed on an inner surface of a first one of the pair of insertion rods; at least one light receiver formed on the inner surface of a second one of the pair of insertion rods to receive light from the sensing light source, and the controller may control the sensing light source to emit light when the power switch is on, and may control the therapeutic light source when the power switch is on, a reference time has elapsed, and an amount of light detected by said at least one light receiver is less than or equal to a predetermined reference light amount. In an exemplary embodiment, the controller may control the sensing light source to output light with a predetermined first light intensity when a reference time has not elapsed after the power switch is turned on, an amount of light detected by the light receiver is larger than the reference light amount, and may control the sensing light source to output light with a predetermined second light intensity when the reference time has elapsed after the power switch is turned on and the amount of light detected by the light receiver is less than or equal to the reference light amount. In an exemplary embodiment, the rhinitis therapeutic device may further include a power indicator. The power indicator may include a plurality of LED display elements, and the controller controls the LED display elements to emit light continuously or in a predetermined pattern when the power is on.

An aspect of the present invention provides a control method of a rhinitis therapeutic device, including: a control method of a rhinitis therapeutic device including a main body; a pair of insertion rods coupled to the main body; at least one sensing light source and at least one light receiver formed on inner surfaces of the pair of insertion rods to face each other; and at least one therapeutic light source formed on the pair of insertion rods, including determining whether power is supplied to the main body; allowing the sensing light source to emit light when power is supplied to the main body; and allowing the therapeutic light source to emit light when the amount of light when an amount of light detected by said at least one light receiver is less than or equal to a predetermined reference light amount.

An aspect of the present invention provides a control method of a rhinitis therapeutic device, including: a control method of a rhinitis therapeutic device including a main body; a pair of insertion rods coupled to the main body; at least one sensing light source and at least one light receiver formed on inner surfaces of the pair of insertion rods to face each other; and at least one therapeutic light source formed on the pair of insertion rods, including determining whether power is supplied to the main body; determining whether a predetermined reference time has elapsed after the start of power supply to the main body; allowing the sensing light source to emit light when power is supplied to the main body; and allowing the therapeutic light source to emit light when the amount of light when the predetermined reference time has elapsed and an amount of light detected by said at least one light receiver is less than or equal to a predetermined reference light amount.

An aspect of the present invention provides a rhinitis therapeutic device including: a main body; a pair of insertion rods coupled to the body and configured to be inserted into the nose for therapy; at least one light source formed at ends of the pair of insertion rods; at least one light receiver formed at ends of the pair of insertion rods; and a control unit accommodated in the main body and electrically coupled to said at least one light source and said at least one light receiving unit, the controller controls the light source to emit light with a first intensity when the power is on, and controls the light source to emit light with a second intensity when am amount of light detected by the light receiver is larger than a reference light amount.

An aspect of the present invention provides a rhinitis therapeutic device including: a main body; a pair of insertion rods coupled to the body and configured to be inserted into the nose for therapy; at least one sensing light source formed on an inner surface of a first one of the pair of insertion rods; at least one light receiver formed on the inner surface of a second one of the pair of insertion rods to receive light from the sensing light source; at least one therapeutic light source formed in the pair of insertion rods; a power switch formed on the main body; a light source operation switch formed on the main body; and a controller accommodated in the main body to be coupled to said at least one sensing light source, said at least one light receiver, said at least therapeutic light source, the power switch, and the power source operation switch, wherein the controller controls light outputs of said at least one light source and said at least one therapeutic light source based on at least two values of an output of the power switch, an output of the light receiver, and an output of the light source operation switch.

### [Advantageous Effects]

According to the rhinitis therapeutic device and the rhinitis therapeutic device control method in accordance with the present invention, when the rhinitis therapeutic device is inserted into the nose, the therapeutic light source is made to emit light, thereby reducing the damage of the organs such as the eye caused by the user carelessness.

### [Description of the Drawings]

FIG. 1A illustrates a rhinitis therapeutic device according to an exemplary embodiment of the present invention.
FIG. 1B is a view for describing positions of a sensing light source and a light receiver 109 of a rhinitis therapeutic device according to an exemplary embodiment of the present invention.
FIG. 2A and FIG. 2B illustrate a rhinitis therapeutic device according to an exemplary embodiment of the present invention.
FIG. 3A and FIG. 3B illustrate a rhinitis therapeutic device according to an exemplary embodiment of the present invention.
FIG. 4A illustrates a flowchart for describing an operation of a controller 105 of a rhinitis therapeutic device 100 according to an exemplary embodiment of the present invention.
FIG. 4B illustrates a flowchart for describing an operation of a controller 105 of a rhinitis therapeutic device 100 according to an exemplary embodiment of the present invention.
FIG. 5A illustrates a rhinitis therapeutic device according to an exemplary embodiment of the present invention, and FIG. 5B and FIG. 5C are views for describing a power indicator according to an exemplary embodiment of the present invention.
FIG. 6 illustrates a rhinitis therapeutic device according to an exemplary embodiment of the present invention.
FIG. 7A and FIG. 7B illustrate a rhinitis therapeutic device according to an exemplary embodiment of the present invention.
FIG. 8A and FIG. 8B are views for describing an operation of a rhinitis therapeutic device according to an exemplary embodiment of the present invention.
FIG. 9A to FIG. 9C are views for describing a difference in that a sensing light source 111 and a light receiver 109 are opposed to each other depending on positions of insertion rods 103a and 103b according to an exemplary embodiment of the present invention.
FIG. 10A to FIG. 10C illustrate a direction of light irradiated through a light irradiator attached in an insertion rod of a rhinitis therapeutic device according to an exemplary embodiment of the present invention.
FIG. 11A to FIG. 11F are views for describing various positions of a sensing light source 111 and a light receiver 109 of a rhinitis therapeutic device according an exemplary embodiment of the present invention.

### [Mode for Invention]

The present invention is intended to illustrate the bars, reference to specific embodiments which may have a number of embodiments can be applied to various changes and describes them in detail from the following detailed description. This, however, is by no means to restrict the invention to the specific embodiments, it is to be understood as embracing all included in the spirit and scope of the present invention changes, equivalents and substitutes. In the following description of the present invention, a detailed description of known techniques that may obscure the subject matter of the present invention, a detailed description thereof will be omitted. In addition, the singular terms used in this specification and the claims should generally be construed to mean one or more unless otherwise stated. In various exemplary embodiments, substantially the same constituent elements may be denoted by the same reference numerals and duplicate explanations may be omitted.

Hereinafter, exemplary embodiments of the present invention will be described in more detail with reference to accompanying drawings.

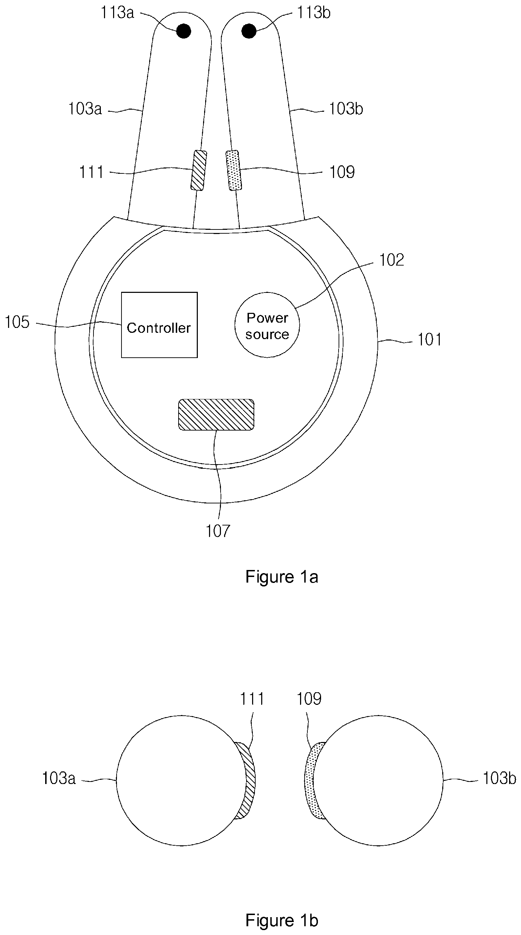

FIG. 1A illustrates a rhinitis therapeutic device according to an exemplary embodiment of the present invention. The rhinitis therapeutic device according to the present exemplary embodiment includes a main body 101, a pair of insertion rods 103a and 103b, a power source 102, a controller 105, a power switch 107, a sensing light source 111, a light receiver109, and at least one therapeutic light source 113a and 113b.

Referring to FIG. 1A, the pair of insertion rods 103a and 103b are coupled to the main body 101. Said at least one therapeutic light source 103a and 103b are formed on the main body 101. Unlike what is illustrated in the drawing, only one therapeutic light source 113a or 113b may be formed, and a plurality of therapeutic light sources 113a and 113b may be formed on various portions of the insertion rods 103a and 103b. According to an exemplary embodiment, the therapeutic light sources 113a and 113b may be laser diodes or LEDs. In addition, when a plurality of therapeutic light sources 113a and 113b are formed, a combination of a laser diode and an LED may be formed. Emission frequencies of the therapeutic light sources 113a and 113b may be partially or entirely the same as or different from each other. According to another exemplary embodiment of the present invention, a frequency of light outputted from the therapeutic light sources 113a and 113b may be controlled depending on an operation of the control unit 105.

As illustrated in FIG. 1A, the power switch 107 is formed at a portion on the main body 101 and is for inputting power on or off of a user. According to another exemplary embodiment of the present invention, the power switch 107 may be formed at a different portion on the main body 101. It will be apparent to those skilled in the art that the power switch 107 may be implemented in various forms such as a pressing operation and a pushing operation. The power switch 107 may also be referred to as another name. For example, it may be referred to as a switch that activates or deactivates a therapeutic action.

FIG. 1B is a view for describing positions of a sensing light source and a light receiver 109 of a rhinitis therapeutic device according to an exemplary embodiment of the present invention.

Referring to FIG. 1A and FIG. 1B, the sensing light source 111 and the light receiver 109 are formed so as to face each other at an inner side of the pair of insertion rods. Accordingly, light outputted from the sensing light source 111 is received by the light receiver 109. According to another preferred embodiment of the present invention, the sensing light source 111 and the light receiver 109 may be formed in plural, rather than one

However, positions where the sensing light source 111 and the light receiver 109 are formed are substantially opposed to the inner surfaces of the pair of insertion rods 103a and 103b. According to the present exemplary embodiment, even when the therapeutic light sources 113a and 113b generate light, a position of the light receiver 109 is adjusted such that the light receiver 109 does not receive the light of the therapeutic light sources 113a and 113b or receive light below the reference amount. According to another exemplary embodiment, even when the therapeutic light sources 113a and 113b generate light, the light receiver 109 may be configured to not receive the light of the therapeutic light sources 113a and 113b by setting a frequency of the light of the therapeutic light sources 113a and 113b be different from that of the light of the sensing light source 111 and allowing the light receiver 109 to receive only light having substantially a same frequency as the light of the sensing light source 111. In addition, the light receiver 109 may be formed to be wider than a light reaching area of the sensing light source 111 in order to prevent an error that the light of the sensing light source 11 is not able to reach the light receiver 109 due to manufacturing tolerance or tolerance in use.

Referring again to FIG. 1A, the power source 102 such as a battery is accommodated in the main body 101, and is electrically coupled to the controller 105. The control unit 105 may be implemented as at least one of various electronic elements such as a microprocessor, a semiconductor chip, and a memory, and is accommodated in the main body 101. According to an exemplary embodiment, although not illustrated in the drawings, a pair of pressing portions are formed on opposite sides of the main body so that when a user presses the pair of pressing portions of the main body 101 from the opposite sides, the pair of insertion rods 103a, 103b are widened so as to be easily inserted into the nose. In addition, an external power source terminal capable of receiving power from the outside may be formed, instead of the power source 102 accommodated in the main body.

The controller 105 is electrically coupled to the power source 102 and/or an external power terminal, the sensing light source 111, the light receiver 109, the therapeutic light sources 113a and 113b, and the power switch 107. When the power switch 107 is turned on, the controller 105 causes the sensing light source 111 to irradiate light. Herein, the meaning that the power switch 107 is turned on indicates that a user starts rhinitis therapy. This indicates that the rhinitis therapy was activated by the user. Hereinafter, the turning-on of the power source means that the rhinitis therapeutics activated by the user operation unless otherwise specified in this specification. Accordingly, the light receiver 109 receives light from the sensing light source 111. The control unit 105 controls the therapeutic light sources 113a and 113b to irradiate light when the light detected by the light receiver 109 is less than or equal to a predetermined reference light amount. That is, when the user intends to perform the therapy using the rhinitis therapeutic device, the power switch 107 is first turned on. When the power switch 107 is turned on to supply power, the sensing light source 111 irradiates light. Thus, the user can visually recognize that the rhinitis therapeutic device is operating by observing that the sensing light source 111 irradiates light. The light irradiated from the sensing light source 111 is detected by the light receiver 109. When the user inserts the pair of insertion rods 103a and 103b into the nose for the therapy, the light from the detection light source 111 is blocked by the nasal septum, thereby making it impossible or difficult to reach the light receiver 109. Accordingly, it is possible to determine whether or not the rhinitis therapeutic device is inserted into the nose depending on the amount of light detected by the light receiver 109. Therefore, the reference light amount may be predetermined so as to distinguish between when the rhinitis therapeutic device is inserted into the nose and when it is not. It will be apparent to those skilled in the art how to determine the reference light amount.

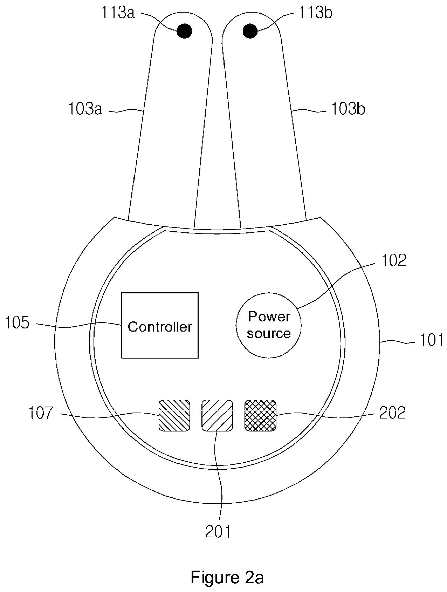

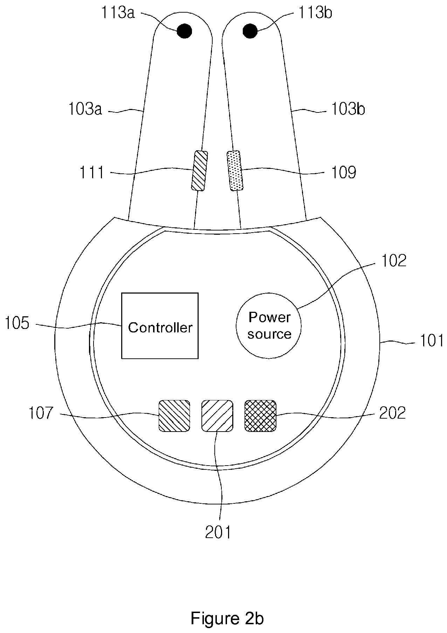

FIG. 2A and FIG. 2B illustrate a rhinitis therapeutic device according to an exemplary embodiment of the present invention. In FIG. 2A and FIG. 2B, substantially the same constituent elements as those in FIG. 1A are denoted by the same reference numerals, and a description thereof will be omitted.

Referring to FIG. 2A, the rhinitis therapeutic device includes a main body 101, a pair of insertion rods 103a and 103b, therapeutic light sources 113a and 113b, a power source 102, a controller 105, a power switch 107, a light source operation switch 201, and a light amount adjustment switch 202.

The power switch 107 is for setting the power on/off in accordance with a user operation. When the power switch 107 is turned on, the control unit 105 controls the therapeutic light sources 113a and 113b to irradiate light less than or equal to a predetermined reference light amount of power source indicator. Thus, the user can visually recognize that the power is normally supplied to the rhinitis therapeutic device. Herein, the predetermined reference light amount of power source indicator is set so that the possibility of damage is low even when the light is temporarily irradiated to the user's eyes by inadvertent use. When the power switch 107 is in an on state, the user inserts the insertion rod of the rhinitis therapeutic device into the nose. Then, when the light source operation switch 201 is turned on by the user, the controller controls the intensity of light outputted to the therapeutic light sources 113a and 113b to be increased to be suitable for rhinitis therapy. That is, when both the power switch 107 and the light source operation switch 201 are on, the controller 105 increases the output of the therapeutic light sources 113a and 113b to control the rhinitis therapy to be performed. The light amount adjustment switch 202 is a switch that can manipulate the output of the therapeutic light sources 113a and 113b to increase or decrease depending on a user's operation. The amount of light may be adjusted stepwise or continuously. According to another exemplary embodiment of the present invention, in the rhinitis therapeutic device illustrated in FIG. 2A, the light amount adjustment switch 202 may be omitted.

Referring to FIG. 2B, a rhinitis therapeutic device according to another exemplary embodiment of the present invention further includes a sensing light source 111 and a light receiver 109 unlike in FIG. 2A.

As illustrated in FIG. 2B, when the power switch 107 is turned on, the controller 105 controls the sensing light source 111 to emit light less than or equal to a predetermined reference light amount. The light outputted from the sensing light source 111 is detected by the light receiver 109. In addition, when the power switch 107 is on, the light source operation switch 201 is on, and an amount of light detected by the light receiver is less than or equal to a reference light amount, the controller 105 controls the therapeutic light sources 113a and 113b. As described above, the sensing light source 111 may be used not only for detecting insertion into the nose but also for the therapy. For this purpose, as a result of determining whether or not the rhinitis therapeutic device is inserted into the nose based on the output of the light receiver 109, when it is inserted into the nose, the output of the sensing light source 111 may be controlled to increase for therapeutic use. As such, the controller may control the output of the sensing light source 111 and/or the output of the therapeutic light sources 113a and 113b based on various combinations of on/off of the power switch 107, on/off of the light source operation switch 201, and the output of the light receiver 109.

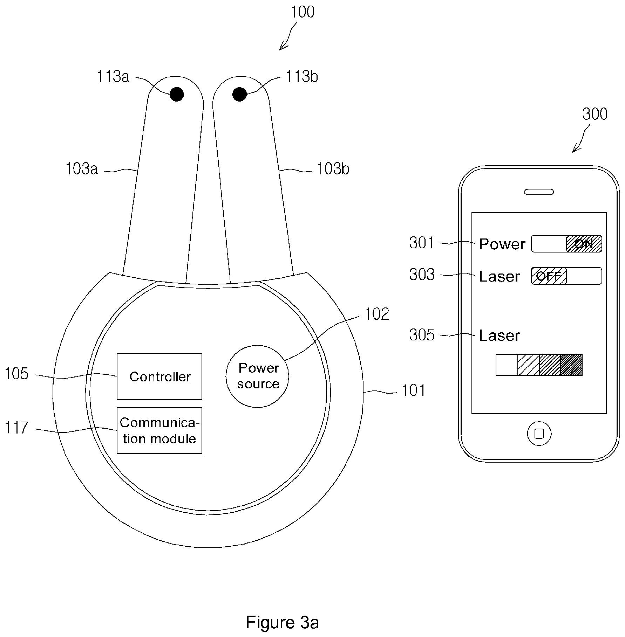

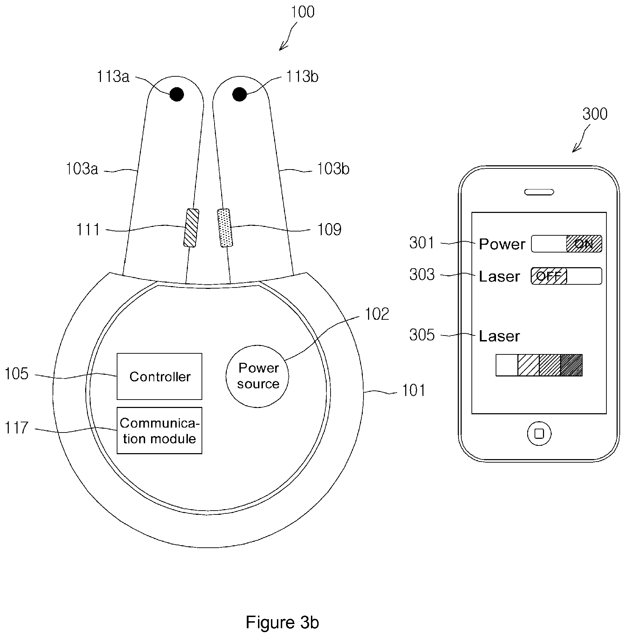

FIG. 3A and FIG. 3B illustrate a rhinitis therapeutic device according to an exemplary embodiment of the present invention. Referring to FIG. 3A and FIG. 3B, the rhinitis therapeutic device 100 further includes a communication module 111, and may transmit and receive a user operation signal from an external device through the communication module 111. The external device 300 may include, e.g., a mobile phone, a notebook, a smart pad, a smart phone, a PC, a remote controller, and the like. According to an exemplary embodiment, the external device 300 includes a power source controller 301. According to another exemplary embodiment, the external device 300 may include a light source controller 302 and/or a light amount controller 303 in addition to the power source controller 301. Herein, the power source controller, 301, the light source controller 302, and the light amount controller 303 of the external device 300 perform substantially the same as functions of the power switch 107, the light source operation switch 201, and the light amount adjustment switch 202. Therefore, as described with reference to FIG. 1A to FIG. 2B, the controller 105 performs control depending on outputs of the power source controller, 301, the light source controller 302, and the light amount controller 303, inputted through the communication module 111.

When the external device 300 is a smartphone or a device that can operate substantially the same as the smartphone, the power source controller 301, the light source controller 302, and the light amount controller 303 may be implemented as an application (usually called an app). Accordingly, a user can download the rhinitis therapeutic device application from the iPhone's App Store or the Android phone's play store, to operate the rhinitis therapeutic device 100.

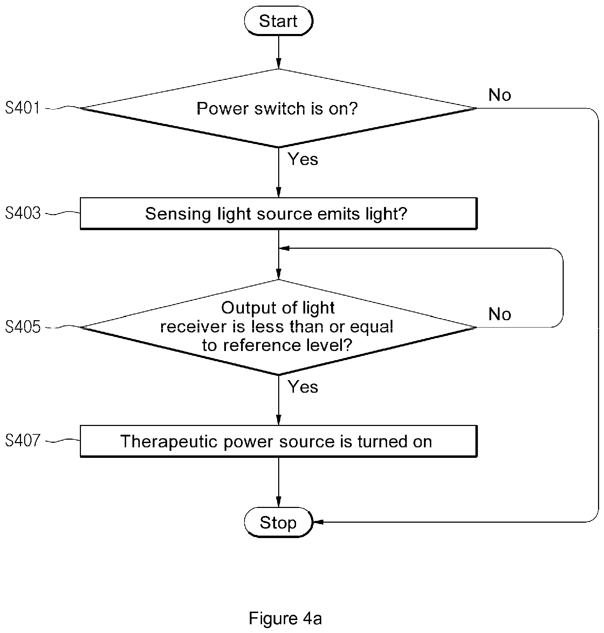

FIG. 4A illustrates a flowchart for describing an operation of a controller 105 of a rhinitis therapeutic device 100 according to an exemplary embodiment of the present invention. Referring to FIG. 4A, it is determined whether the power switch 107 is on or off in step S401. When the power switch 107 is on, power is supplied to the sensing light source 111 so as to emit light in step S403. In step S405, it is determined whether the output of the light receiver 109 is less than or equal to the reference light amount. When the determination result of step S405 is YES, power is supplied to the therapeutic light sources 113a and 113b to emit light in step S407. After the therapy is finished, the power is turned off.

A therapy time may be terminated by the user operating the power switch 107 and/or the power source controller 301, or may be automatically terminated when a predetermined therapy time has elapsed. For example, values such as 5 minutes, 10 minutes, 15 minutes, and 20 minutes may be preset in the rhinitis therapeutic apparatus 100, or the therapy time may be set by the user. The setting of the therapy time may be performed through the external device 300.

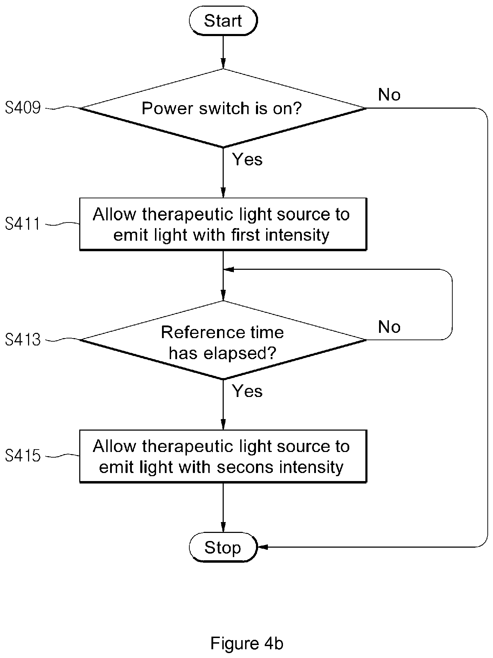

FIG. 4B illustrates a flowchart for describing an operation of a controller 105 of a rhinitis therapeutic device 100 according to an exemplary embodiment of the present invention. Referring to FIG. 4B, in step S409, it is determined whether the value of the power switch 107 or the power source controller 301 inputted through the communication module 117 is on.

When the power is on, the therapeutic light sources 113a and 113b are caused to emit light with a first intensity in step S411. The first intensity is set to a relatively low intensity in order to prevent eye damage even when light is irradiated to the eye due to carelessness of the user. When the therapeutic light sources 113a and 113b emit light with the first intensity, there is an effect of visually informing the user that the power is supplied. The user may confirm the power supply and insert the rhinitis therapeutic device 100 into the nose. Herein, it is necessary to predict and specify a usual time for the user to confirm the power supply and to insert the rhinitis therapeutic device 100 into the nose. In step S413, it is determined whether or not a predetermined reference time has elapsed in consideration of this point. When the reference time has elapsed, the therapeutic light sources 113a and 113b are caused to emit light with a second intensity in step S415. Herein, the second intensity is higher than the first intensity, and it is necessary to set the intensity to be suitable for the rhinitis therapy. In addition, the second intensity of the therapeutic light sources 113a and 113b may be adjusted by user operation, that is, by the light amount control switch 202 and the light amount controller 303.

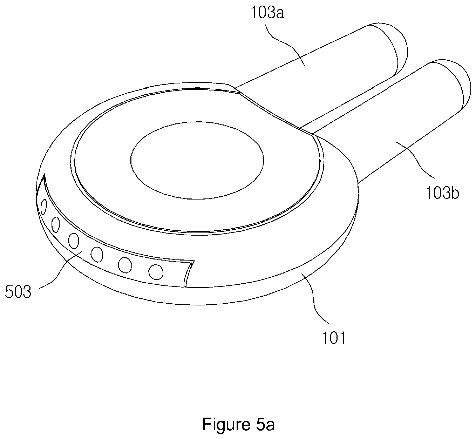

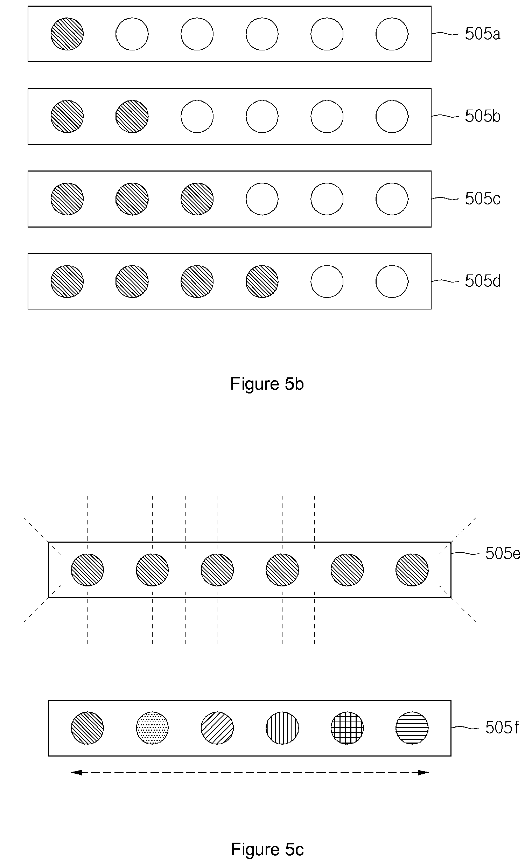

FIG. 5A illustrates a rhinitis therapeutic device according to an exemplary embodiment of the present invention, and FIG. 5B and FIG. 5C are views for describing a power indicator according to an exemplary embodiment of the present invention. Referring to FIG. 5A to FIG. 5C, a power indicator 503 is formed in the main body 101 of the rhinitis therapeutic device 100. According to an exemplary embodiment, the power indicator 503 includes at least one LED. The power indicator 503 is used to indicate to the user whether power is being supplied to the rhinitis therapeutic device 100 and which operation state it is in. When the therapeutic light sources 113a and 113b are emitting light, the user needs to pay attention such that the light is not irradiated by the user's eyes. Therefore, the rhinitis therapeutic apparatus 100 can inform the user of the operation state through the power indicator 503 and protect the user from unintended light irradiation, thereby preventing eye damage. FIG. 5B illustrates an example of an operation of the display unit 401. When the power switch is turned on, a first LED indicator lamp is turned on (505a). When the light source operation switch is turned on, a second LED indicator lamp is turned on together (505b). The LED indicator lamps may be turned on one by one for each step in at least one step, depending on the intensity of the light source (505c and 505d). FIG. 5C illustrates another example of the operation of the display unit 401. In a process of illuminating the light, all the LED indicator lamps flash (505e), or the LED indicator lamps are turned on and off repeatedly sequentially from left to right or right to left. FIG. 5B and FIG. 5C illustrate some of the display forms of the power indicator 503, and it will be apparent to those skilled in the art to display a power supply status, an operation mode, and the like in various ways by using LED lighting, LED lighting pattern (e.g., a flickering pattern over time). Herein, the operation mode may include various operation modes for checking whether the therapeutic light sources 113a and 113b irradiate light, whether the intensity of the light of the therapeutic light sources 113a and 113b is in a ready state (emitting light with the first intensity) or in a therapeutic state, whether the therapeutic light sources 113a and 113b irradiate therapeutic light, and the like, which are described above.

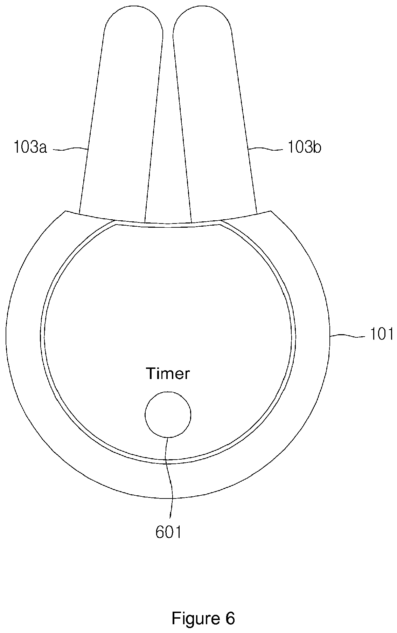

FIG. 6 illustrates a rhinitis therapeutic device according to an exemplary embodiment of the present invention. Referring to FIG. 6, the rhinitis therapeutic apparatus 100 includes a timer 601. In the case where the user has the timer 601 that can be operated by the user, the user can adjust the reference time and/or the therapy time used by the controller 105.

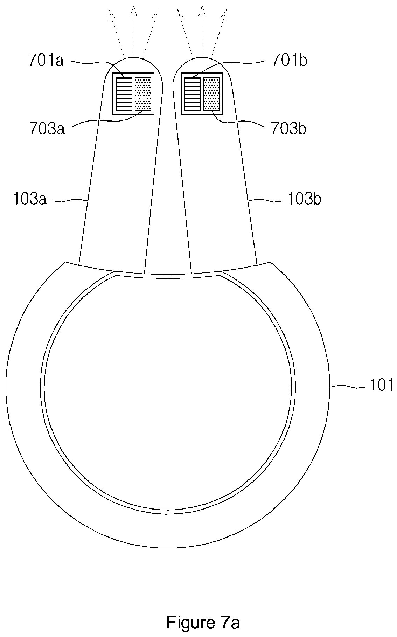

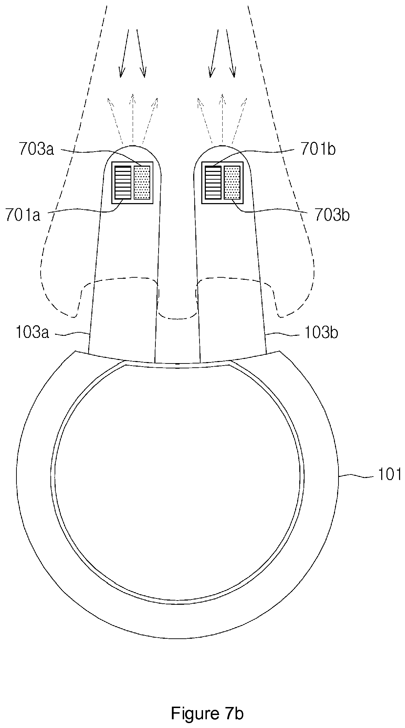

FIG. 7A and FIG. 7B illustrate a rhinitis therapeutic device according to an exemplary embodiment of the present invention. Light irradiators 701a and 701b and light receivers 703a and 703b are formed at ends of the pair of insertion rods. When the power is supplied, the light irradiator 701a outputs light less than or equal to the reference light amount through a slit or a hole in the ready state. The light outputted at this time is preferably light that does not damage or hardly damage the user's eyes. With no insertion rods inserted in the nose, the light irradiated through the slit or hole will generally go straight infinitely into the air. Accordingly, the light receivers 701 and 703b installed at the ends of the insertion rods cannot sense light. However, after the user inserts the insertion rods into the nose for the rhinitis therapy, the light is reflected through of a skin etc., in the nose. Accordingly, the light receivers 701b and 703b can sense light of a specific wavelength. The controller controls the light irradiator 701a and 701b to irradiate the therapeutic light when the light receivers 701b and 703b sense light of a specific wavelength. Although not illustrated in the drawing, a laser diode and/or an LED may be attached to the light irradiator.

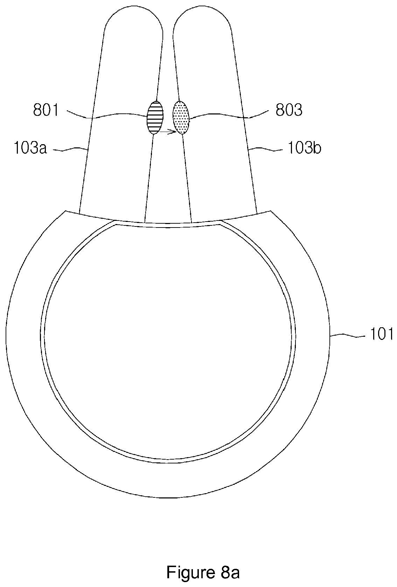

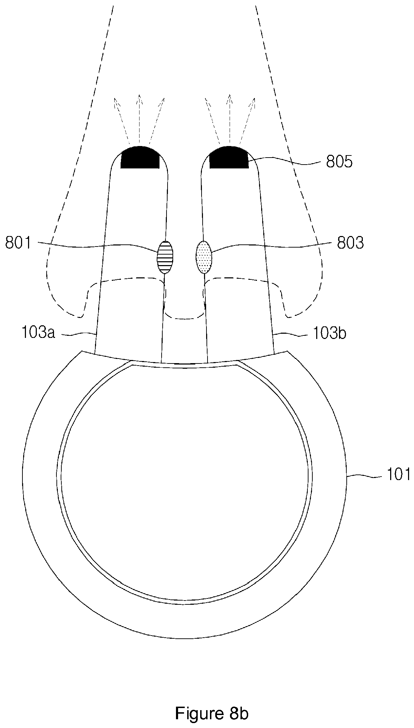

FIG. 8A and FIG. 8B are views for describing an operation of a rhinitis therapeutic device according to an exemplary embodiment of the present invention. Referring to FIG. 8A and FIG. 8B, a light irradiator 801 and a light receiver 803 are formed inside the insertion rods. When power is supplied, the light irradiator 801 irradiates light and the light output from the light irradiator 801 is detected by the light receiver 803. When an amount of the light detected by the light receiver 803 is larger than or equal to the reference light amount, the controller controls the actual therapeutic light 805 not to be irradiated. Thus, it is possible to prevent eye damage due to unintended light irradiation. When the user inserts the insertion rods into the nose for the rhinitis therapy, the light path is blocked by the nasal septum, so no light or light less than or equal to the reference light amount is detected in the light receiver 803. Therefore, the controller controls the therapeutic light 805 to be irradiated when the amount of the detected light is less than or equal to the reference light amount. The light irradiator may include at least one light source, e.g., a laser diode and/or an LED. Further, at least one slit or hole may be formed in the light irradiator such that light is irradiated through the slit or hole.

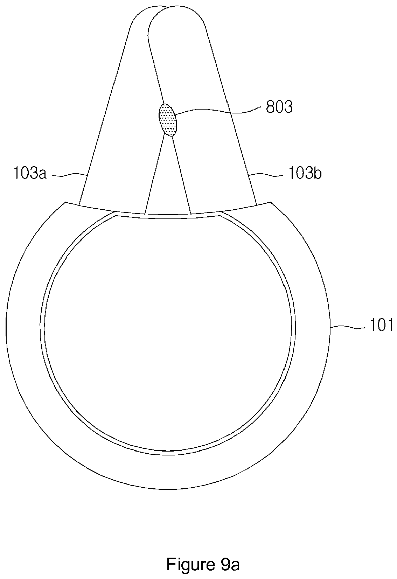

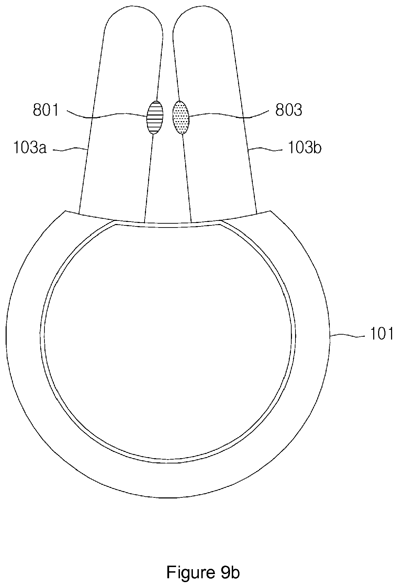

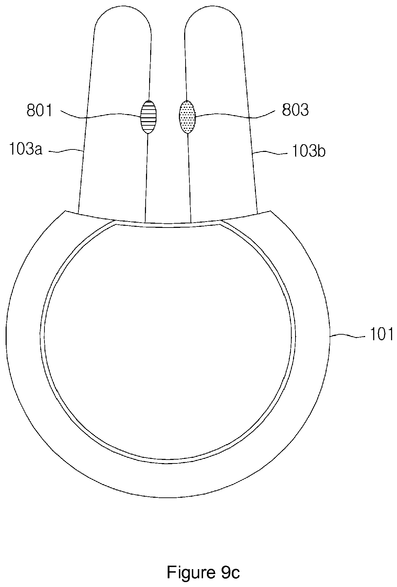

FIG. 9A to FIG. 9C are views for describing a difference in that a sensing light source 111 and a light receiver 109 are opposed to each other depending on positions of insertion rods 103a and 103b according to an exemplary embodiment of the present invention. When the insertion rods have a clamp structure, a position thereof is changed depending on force applied to the clamp structure, thereby causing a deviation from the sensing light source 111 and the light receiver 109 facing each other

Accordingly, in spite of such a deviation, it is necessary to irradiate the light in such a predetermined range so as to enable the light output from the sensing light source 111 to be detected by the light receiver 109

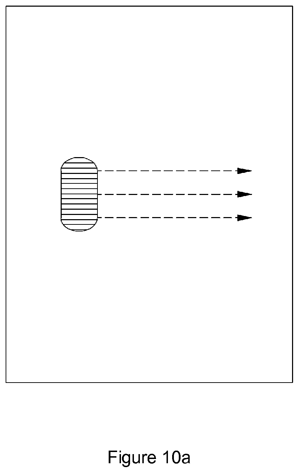

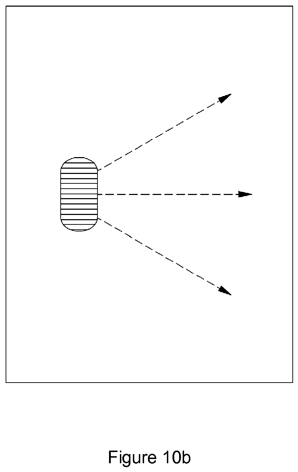

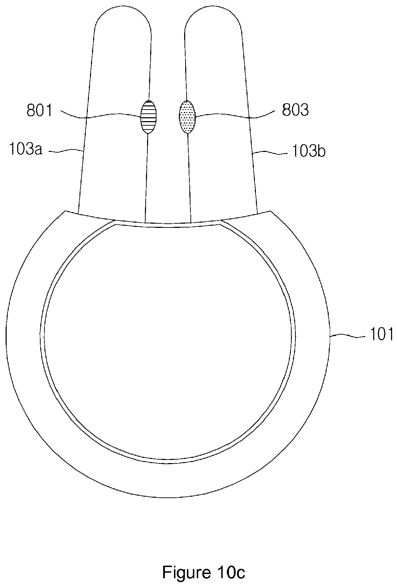

FIG. 10A to FIG. 10C illustrate a direction of light irradiated through a light irradiator attached in an insertion rod of a rhinitis therapeutic device according to an exemplary embodiment of the present invention. The light irradiated through the light irradiator may be irradiated in the form of a straight line (FIG. 10A). Further, the light irradiated through the light irradiator may be irradiated in one or more directions (FIG. 10B and FIG. 10C). This is to allow the light receiver 803 to detect the light from the sensing light source 111 even when the positions of the insertion rods are changed.

Further, the light outputted from the sensing light source 111 may be irradiated in an oblique direction and/or in a direction (transverse direction) that is perpendicular to a longitudinal direction of the insertion rods. This is to enable the light receiving unit 109 to detect the light outputted from the sensing light source 111 even when the pair of insertion rods are mechanically spaced from each other in the manufacturing of the rhinitis therapeutic device 100.

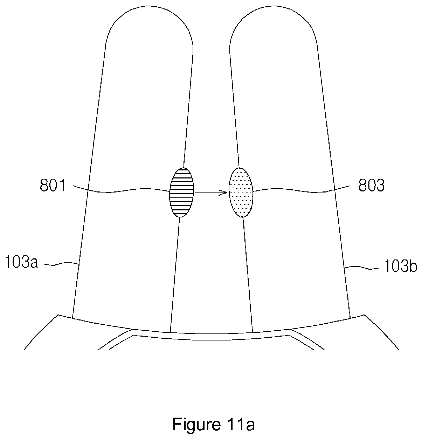

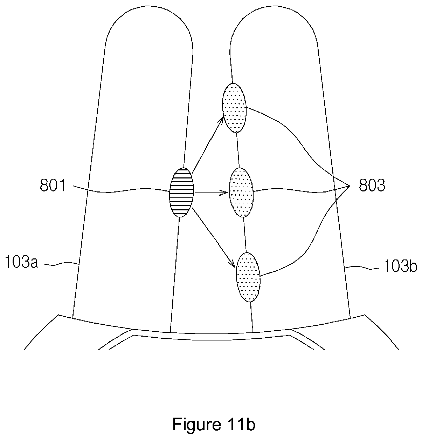

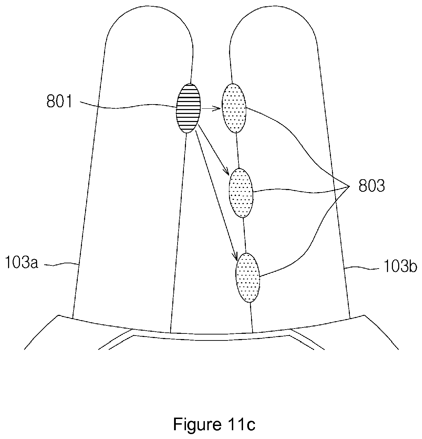

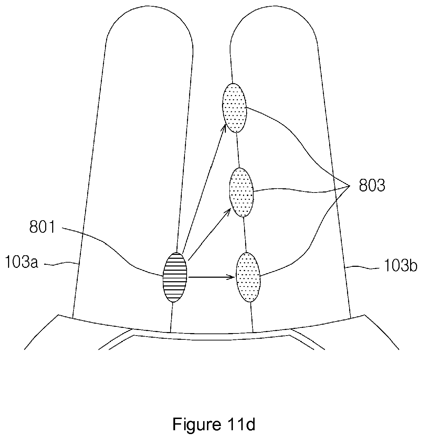

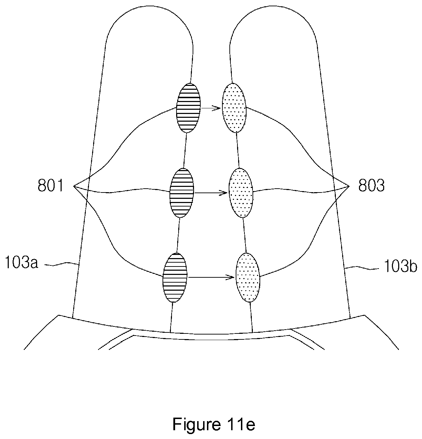

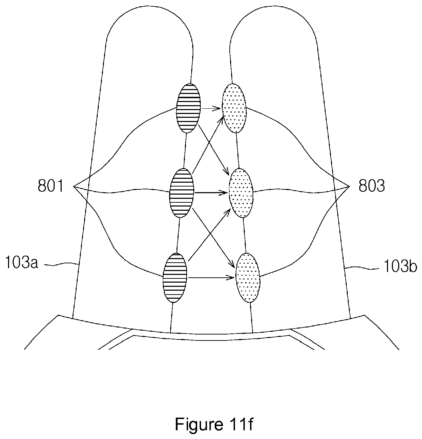

FIG. 11A to FIG. 11F are views for describing various positions of a sensing light source 111 and a light receiver 109 of a rhinitis therapeutic device according an exemplary embodiment of the present invention. FIG. 11A illustrates the sensing light source 111 and the light receiver 109 which are respectively formed on inner surfaces of the insertion rods so as to face each other in a one-to-one correspondence with each other. FIG. 11B to FIG. 11D illustrate examples in which one sensing light source 111 is formed on an inner surface of one of the pair of insertion rods, and a plurality of, e.g., three, light receivers 109 is formed on an inner surface of the other insertion rod. Herein, the positions of the three light receivers 109 may be slightly shifted in a longitudinal direction of the insertion rods and/or in a direction perpendicular to the insertion rods, so as to correct misalignment of the insertion rods occurring in the manufacturing and/or misalignment in use. FIG. 11E and FIG. 11F illustrate examples in which a plurality of sensing light sources 111 are formed inside on an inner surface of the pair of insertion rods, and a plurality of light receivers 109 are formed on an inner surface of the other insertion rod. In this case as well, the positions of the sensing light sources 111 and the light receivers 109 may be formed by dispersing the insertion rods in the longitudinal direction and/or the perpendicular direction to the insertion rods in order to correct manufacturing deviation and/or deviation in use.

In the foregoing, the exemplary embodiments of the present invention have been described. One of ordinary skill in the art will appreciate that the invention may be implemented without departing from the essential characteristics of the invention in a modified form. The exemplary embodiments should be considered in a descriptive sense only and not for purposes of limitation. The scope of the invention, not by the detailed description given in the appended claims, and all differences within the equivalent scope will be construed as being included in the present invention.

## Claims

1. A rhinitis therapeutic device comprising:
a power source;
a main body configured to accommodate the power source;
a pair of insertion rods coupled to the body and configured to be inserted into the nose for therapy;
at least one sensing light source;
at least one light receiver;
at least one therapeutic light source formed in the pair of insertion rods; and
a controller accommodated in the main body to be coupled to the power source, said at least one sensing light source, said at least one light receiver, and said at least therapeutic light source,
said at least one sensing light source is formed on an inner surface of a first one of the pair of insertion rods, and said at least one light receiver is formed on an inner surface of a second one of the pair of insertion rods, to correspond to said at least one sensing light source to receive light from the sensing light source, and
the controller controls the sensing light source to emit light when the power source is on, and controls said at least one therapeutic light source to emit light when the power is on and an amount of light detected by the light receiver is less than or equal to a predetermined reference light amount.

2. The rhinitis therapeutic device of claim 1, wherein
said at least one therapeutic light source includes at least one laser diode.

3. The rhinitis therapeutic device of claim 1, wherein
said therapeutic light source includes at least one LED.

4. The rhinitis therapeutic device of claim 2 or claim 3, wherein
the sensing light source outputs light with a predetermined first light intensity when the amount of light detected by the light receiver is less than or equal to the reference light amount and outputs light with a predetermined light intensity when the amount of light detected by the light receiver is larger than the reference light amount, and the first light intensity is lower than the second light intensity.

5. The rhinitis therapeutic device of claim 4, further comprising
a power switch for turning the power source on/off.

6. The rhinitis therapeutic device of claim 4, further comprising
a communication module wherein a signal for turning on/off the power source is received from an external device through the communication module, and is transmitted to the controller.

7. The rhinitis therapeutic device of claim 4, further comprising
a power indicator formed at a side of the main body opposite to a side at which the pair of insertion rods are formed,
the power indicator includes a plurality of LED display elements, and
the controller controls the LED display elements to emit light continuously or in a predetermined pattern when the power is on.

8. The rhinitis therapeutic device of claim 4, wherein
light output from the sensing light source is emitted in an extended form obliquely and/or in a direction that is substantially perpendicular to a longitudinal direction of the pair of insertion rods.

9. A rhinitis therapeutic device comprising:
a main body;
a power switch formed on the main body to turn on/off the power by a user operation;
a pair of insertion rods coupled to the body and configured to be inserted into the nose for therapy;
at least one therapeutic light source formed in the pair of insertion rods; and
a control unit accommodated in the main body and coupled to said at least one therapeutic light source,
wherein the controller receives an on signal from the power switch and then controls said at least one therapeutic light source to emit light after a predetermined reference time elapses.

10. The rhinitis therapeutic device of claim 9, wherein
said at least one therapeutic light source includes at least one laser diode.

11. The rhinitis therapeutic device of claim 9, wherein
said at least one therapeutic light source includes at least one LED.

12. The rhinitis therapeutic device of claim 10 or claim 11, further comprising:
at least one sensing light source formed on an inner surface of a first one of the pair of insertion rods;
at least one light receiver formed on the inner surface of a second one of the pair of insertion rods to receive light from the sensing light source,
the controller controls the sensing light source to emit light when the power switch is on, and controls the therapeutic light source when the power switch is on, a reference time has elapsed, and an amount of light detected by said at least one light receiver is less than or equal to a predetermined reference light amount.

13. The rhinitis therapeutic device of claim 12, wherein
the controller
controls the sensing light source to output light with a predetermined first light intensity when a reference time has not elapsed after the power switch is turned on, an amount of light detected by the light receiver is larger than the reference light amount, and controls the sensing light source to output light with a predetermined second light intensity when the reference time has elapsed after the power switch is turned on and the amount of light detected by the light receiver is less than or equal to the reference light amount.

14. The rhinitis therapeutic device of claim 9, wherein
a power indicator formed at a side of the main body opposite to a side at which the pair of insertion rods are formed,
the power indicator includes a plurality of LED display elements, and
the controller controls the LED display elements to emit light continuously or in a predetermined pattern when the power is on.

15. A control method of a rhinitis therapeutic device including a main body; a pair of insertion rods coupled to the main body; at least one sensing light source and at least one light receiver formed on inner surfaces of the pair of insertion rods to face each other; and at least one therapeutic light source formed on the pair of insertion rods, the control method comprising:
determining whether power is supplied to the main body;
allowing the sensing light source to emit light when power is supplied to the main body; and
allowing the therapeutic light source to emit light when the amount of light when an amount of light detected by said at least one light receiver is less than or equal to a predetermined reference light amount.

16. A control method of a rhinitis therapeutic device including a main body; a pair of insertion rods coupled to the main body; at least one sensing light source and at least one light receiver formed on inner surfaces of the pair of insertion rods to face each other; and at least one therapeutic light source formed on the pair of insertion rods, the control method comprising:
determining whether power is supplied to the main body;
determining whether a predetermined reference time has elapsed after the start of power supply to the main body;
allowing the sensing light source to emit light when power is supplied to the main body; and
allowing the therapeutic light source to emit light when the amount of light when the predetermined reference time has elapsed and an amount of light detected by said at least one light receiver is less than or equal to a predetermined reference light amount.

17. A rhinitis therapeutic device comprising:
a main body;
a pair of insertion rods coupled to the body and configured to be inserted into the nose for therapy;
at least one light source formed at ends of the pair of insertion rods;
at least one light receiver formed at ends of the pair of insertion rods; and
a control unit accommodated in the main body and electrically coupled to said at least one light source and said at least one light receiving unit,
wherein the controller controls the light source to emit light with a first intensity when the power is on, and controls the light source to emit light with a second intensity when am amount of light detected by the light receiver is larger than a reference light amount.

18. A rhinitis therapeutic device comprising:
a main body;
a pair of insertion rods coupled to the body and configured to be inserted into the nose for therapy;
at least one sensing light source formed on an inner surface of a first one of the pair of insertion rods;
at least one light receiver formed on the inner surface of a second one of the pair of insertion rods to receive light from the sensing light source;
at least one therapeutic light source formed in the pair of insertion rods;
a power switch formed on the main body;
a light source operation switch formed on the main body; and
a controller accommodated in the main body to be coupled to said at least one sensing light source, said at least one light receiver, said at least therapeutic light source, the power switch, and the power source operation switch,
wherein the controller controls light outputs of said at least one light source and said at least one therapeutic light source based on at least two values of an output of the power switch, an output of the light receiver, and an output of the light source operation switch.
